# EUROPÄISCHE PATENTANMELDUNG

(11) **EP 0 880 947 A2**
(43) Veröffentlichungstag der Anmeldung: **02.12.1998**
(21) Anmeldenummer: 98250169.4
(22) Anmeldetag: 18.05.1998
(51) Int. Cl.: A61F 2/06

(54) **Stent**

(30) Priorität: 23.05.1997 DE 19722857
(71) Anmelder: BIOTRONIK Mess- und Therapiegeräte GmbH & Co Ingenieurbüro Berlin, D-12359 Berlin (DE)
(72) Erfinder: Kranz, Curt, Dr.-Ing., 10825 Berlin (DE)
(74) Vertreter: Christiansen, Henning, Dipl.-Ing.

(57) **Zusammenfassung**

Stent, insbesondere Koronarstent, bestehend aus mindestens einem dünnwandigen, rohrförmigen Körper (2), dessen Mantelfläche (1) in stegartige, sich in Längsrichtung des Stents erstreckende und in Umfangsrichtung des Stents expansible Elemente (4, 4', 4", 4"', 4"") unterteilt ist, die über jeweils wenigstens einen Verbindungssteg (6, 6') mit den beiden in Umfangsrichtung des Stents benachbarten expansiblen Elementen (4, 4', 4", 4"', 4"") und über einen stirnseitigen Anschluß (8) mit der Stirnseite (4.4') eines in Längsrichtung des Stents angrenzenden expansiblen Elements (4, 4', 4", 4"', 4"") verbunden sind, wobei die Mantelfläche (1) im nicht expandierten Zustand im wesentlichen frei von Ausnehmungen ist und durch Trennfugen (3) unterteilt ist, die im Verhältnis zur Stegbreite der expansiblen Elemente (4, 4', 4", 4"', 4"") eine wesentlich geringere Breite aufweisen, sowie Verfahren zu seiner Herstellung.

## Beschreibung

Die Erfindung betrifft einen Stent, insbesondere Koronarstent, als intraluminales Expansionselement, entsprechend der im Oberbegriff des Anspruchs 1 genannten Art sowie ein Verfahren zu dessen Herstellung.

Aus den europäischen Patentschriften EP 0 364 787 B1 und EP 335 341 B1 ist ein aufweitbares intraluminales Element mit mindestens einem dünnwandigen, rohrförmigen Teil (nachfolgend als Stent bezeichnet) bekannt. Die Mantelfläche des Stents ist durchbrochen netzförmig ausgebildet und weist dabei Ausnehmungen auf, die durch sich geradlinig in axialer und in Umfangsrichtung erstreckende stegartige Elemente von geringer Materialstärke begrenzt sind. Die stegartigen, in Umfangsrichtung des Stents expansiblen Elemente bestehen aus der restlichen Rohrwandung, von der das Material im Bereich der Ausnehmungen entfernt wurde.

Ein weiterer ebenfalls netzförmig aufgebauter rohrförmiger Stent ist aus der internationalen Patentanmeldung WO 96 03 092 A1 bekannt. Der Stent ist aus sich teils in Umfangsrichtung, teils in Längsrichtung des Stents erstreckenden, mäanderförmig angeordneten Stegen aufgebaut. Die Mantelfläche des Stents ist ebenfalls durchbrochen ausgebildet und weist zwischen den Stegen Ausnehmungen auf in deren Bereich das Material der aus der Rohrwandung entfernt wurde

Derartige Stents werden in einem operativen Eingriff unter Einwirkung von von innen nach außen gerichteten Kräften durch einen mit Druckgas beaufschlagten schlauchförmigen Dilator, expandiert. Der Stent behält dabei trotz Verformung seine Rohrform bei und weitet das durch Ablagerungen verengte Gefäß auf.

Aus Sicherheitsgründen bleibt die Verformung der Stege weit unterhalb eines möglichen Gefahrenbereichs, da der Bruch eines Steges dazu führen würde, daß dessen freie Enden im Bereich der Bruchstelle in das Innere des mit dem Stent versehenen Gefäßes hineinragen würde. Durch die damit verbundene Gefahr der Bildung von Restenosen würde nicht nur der Erfolg des Eingriffs selbst in Frage gestellt, sondern auch das Leben des Patienten gefährdet.

Derartige dünne Netzstrukturen sind aber schon während des gesamten Zeitraumes von ihrer Herstellung bis zu ihrer Verwendung sehr empfindlich gegenüber mechanischen Beschädigungen durch Krafteinwirkung, die zu ihrer Unbrauchbarkeit führen können. Eine derartige Beschädigung kann im nicht expandierten Zustand unter Umständen auch nicht zu erkennen sein und mit den geschilderten nachteiligen Auswirkungen beispielsweise erst beim Expandieren des Stents zum endgültigen Bruch eines oder mehrerer Stege führen

Die bekannten Stents weisen weiterhin den Nachteil auf, daß die Herstellung der netzartigen Struktur des Stents sehr zeitintensiv und mit einem hohen Aufwand für das Entfernen des aus der Mantelfläche herausgeschnittenen Materials verbunden ist.

Ausgehend von den Mängeln des Standes der Technik liegt der Erfindung die Aufgabe zugrunde, einen expandierbaren Stent der eingangs genannten Gattung anzugeben, welcher bei einfacher Herstellbarkeit eine auch im nicht expandierten Zustand gute Stabilität aufweist, sowie ein Verfahren zu dessen Herstellung anzugeben.

Die Aufgabe wird durch einen Stent mit den Merkmalen des Anspruchs 1 sowie ein Verfahren mit den Merkmalen des Anspruchs 26 gelöst.

Die Erfindung schließt die technische Lehre ein, daß bei einen gattungsgemäßen Stent sowohl der Herstellungsaufwand als auch die Beschädigungsempfindlichkeit bedeutend reduziert werden kann, wenn die Mantelfläche des rohrförmigen Ausgangskörpers im nicht expandierten Zustand des Stents im wesentlichen frei von Ausnehmungen ist und durch lediglich schmale Trennfugen in expansible Elemente und deren Verbindungen untereinander unterteilt ist.

Hierdurch wird zum einen der Vorteil erreicht, daß sich der Schnittaufwand erheblich reduziert, da beispielsweise weniger und kürzere Schnitte in der Mantelfläche vorgenommen werden müssen, um die unterteilenden Trennfugen zu erzeugen. Zum anderen muß bei der Herstellung bedeutend weniger Material entfernt werden, wodurch sowohl der Aufwand für das Austragen des Materials als auch der Anfall von zu entsorgendem Abfall reduziert wird.

Ein weiterer Vorteil liegt darin, daß die im nicht expandierten Zustand im wesentlichen ausnehmungsfreie Mantelfläche des Stents weit unempfindlicher gegen mechanische Beschädigungen ist als die bisherigen netzartigen Stents, da Kräfte großflächig in die Stentstruktur eingeleitet werden und sich nicht auf einzelne, dünne Bestandteile einer netzartigen Struktur konzentrieren. Eine Beschädigung des Stents durch mechanische Belastungen bei Herstellung, Lagerung, Transport und auch vor und während der Operation wird durch bedeutend unwahrscheinlicher.

Ein weiterer Vorteil des erfindungsgemäßen Stents liegt darin, daß er im expandierten Zustand einen wesentlich größeren Teil der Gefäßwand bedeckt, als dies bei den bekannten netzartig strukturierten Stents der Fall ist. Die für erneute Ablagerungen zur Verfügung stehende Gefäßwandfläche wird dadurch erheblich reduziert, so daß auch das Risiko der Bildung von Restenosen weiter vermindert wird.

Das Erfindungsmerkmal, daß die Mantelfläche im wesentlichen frei von Ausnehmungen ist, ist dabei so zu verstehen, daß ein wesentlicher Anteil der Mantelfläche eine geschlossene lediglich durch Trennfugen unterteilte Oberfläche aufweist. Es können jedoch, beispielsweise innerhalb der einzelnen expansiblen Elemente, kleinere Aussparungen vorgesehen sein, deren Gesamtheit auf die Mantelfläche bezogen jedoch nicht wesentlich ins Gewicht fällt.

Bei einer bevorzugten Ausführung des erfindungsgemäßen Stents weisen die Trennfugen eine Breite auf, die im wesentlichen der Breite eines sauberen Schnittspaltes beim Durchtrennen der Mantelfläche mittels eines Schneidstrahls, beispielsweise eines Schneid-Wasserstrahls, insbesondere aber eines Laserstrahls entspricht. Durch diese besonders schmalen Trennfugen wird eine besonders hohe Stabilität des nicht expandierten Stents erzielt. Vorzugsweise weisen die Trennfugen daher eine Breite auf, die im wesentlichen der erzielbaren minimalen Breite eines sauberen Schnittspaltes beim Durchtrennen der Mantelfläche mittels eines Schneidstrahls, insbesondere eines Laserstrahls entspricht. Hierdurch wird nicht nur dank der geringen Trennfugenbreite eine hohe Stabilität des Stents erreicht. Es kann zudem eine Verringerung der Herstellungszeit des Stents erzielt, da die Breite eines sauberen Schnittspaltes unmittelbar mit der Vorschubgeschwindigkeit des Schneidstrahls zusammenhängt. Die minimalen Breite der Trennfugen wird gerade bei der maximalen Vorschubgeschwindigkeit des Schneidstrahls erzielt, bei der noch ein sauberer Schnittspalt beim Durchtrennen der Mantelfläche mittels des Schneidstrahls entsteht.

Bei einer weiteren bevorzugten Ausführung des erfindungsgemäßen Stents verlaufen die Verbindungsstege sowohl zur Längs- als auch zur Umfangsrichtung des Stents geneigt und sind zwischen benachbarten expansiblen Elementen im wesentlichen über ihre gesamte Länge seitlich jeweils von der Trennfuge begrenzt sind, die in diesem Bereich die Außenkontur des jeweiligen expansiblen Elements begrenzt. Hierdurch wird in einfacher Weise erreicht, daß zwischen den Längsseiten der einzelnen expansiblen Elemente keine Lükken, d.h. Aussparungen entstehen, zum anderen wird erreicht, daß mit einer einzigen Trennfuge sowohl ein Teil der Kontur des expansiblen Elements als auch die Hälfte der Außenkontur des Verbindungssteges zum angrenzenden expansiblen Element definiert ist, wodurch sich der Herstellungsaufwand reduziert.

Bei einer vorteilhaften Weiterbildung des erfindungsgemäßen Stents ist die Außenkontur der expansiblen Elemente zu ihrer Längsachse symmetrisch. Sie weist dabei einen sich sowohl in Längs- als auch in Umfangsrichtung erstreckenden flächigen ersten Abschnitt sowie einen sich wenigstens im wesentlichen in Umfangsrichtung erstreckenden zweiten Abschnitt auf, die durch einem sich im wesentlichen in Längsrichtung erstreckenden Zwischenabschnitt verbunden sind. Diese Ausbildung der expansiblen Elemente ermöglicht eine besonders platzsparende, dichtgedrängte Anordnung der expansiblen Elemente, mit reduzierten Zwischenräumen zwischen den Elementen. So können die expansiblen Elemente beispielsweise so ausgebildet und angeordnet sein, daß der zweite Abschnitt eines Elementes in die durch den Zwischenabschnitt gebildeten Einschnürung zwischen dem ersten und zweiten Abschnitt des benachbarten Elementes hineinragt, wodurch sich ein geringer Abstand zwischen den beiden Elementen ergibt. Mögliche Aussparungen in der Mantelfläche werden dadurch weiter reduziert.

Die einzelnen Abschnitte, insbesondere die ersten und zweiten Abschnitte sind dabei vorzugsweise aus regelmäßige Formen, wie beispielsweise Kreisen, Ellipsen, Rechtecken, Quadraten, Vielecken oder aus aus diesen zusammengesetzten bzw. an diese angenäherten Gebilden erzeugt.

Bei einer besonders günstigen Ausführung des erfindungsgemäßen Stents sind zwei in Umfangsrichtung des Stents aneinandergrenzende expansible Elemente jeweils um 180° zueinander gedreht angeordnet. Dabei greift jeweils der erste Abschnitt des einen Elements in den Zwischenraum zwischen dem ersten und zweiten Abschnitt des anderen Elements ein. Weiterhin ist der Verbindungssteg zwischen den beiden ersten Abschnitten angeordnet und die einander unmittelbar zugewandten Außenkonturen der beiden Elemente sind durch dieselben Trennfugen begrenzt. Hierdurch wird in vorteilhafter Weise zum einen der Herstellungsaufwand des Stents reduziert, da mit einer Trennfuge Teile der Außenkontur zweier aneinandergrenzender Elemente definiert sind, wodurch sich die Anzahl und Länge der zu erstellenden Trennfugen verringert. Weiterhin wird erreicht, daß zwischen den in Umfangsrichtung des Stents aneinandergrenzenden expansiblen Elemente keine nennenswerten Aussparungen entstehen, da sich die expansiblen Elemente quasi lückenlos aneinanderschmiegen und der Verbindungssteg die noch verbleibende Lücke zwischen den beiden Elementen ausfüllt. Die Stirnseite der zweiten Abschnitte ist dabei dann vorzugsweise gerade in Umfangsrichtung verlaufend ausgebildet, um den lückenlosen Anschluß weiterer expansibler Elemente in Längsrichtung des Stents zu ermöglichen.

Eine vorteilhafte Weiterbildung weist zur Ausbildung eines entlang der Außenkontur umlaufenden Steges geringer Breite im Innern der expansiblen Elemente eine innere Trennfuge auf, die zumindest abschnittsweise in im wesentlichen konstanten Abstand von der Außenkontur verläuft. Hierdurch entstehen auf besonders einfache Weise in Umfangsrichtung expansible Elemente. Der umlaufende Steg, an dem die Verbindungselemente angeschlossen sind, kann sich bei der Expansion des Stents nach Art der bekannten stegförmigen expansiblen Elemente verformen und stellt so die gewünschte Funktion des Stents sicher. Der Steg weist dabei zumindest abschnittsweise - beispielsweise in den Bereichen der einzelnen Abschnitte des Elements - konstante Breite auf, um bei der Expansion eine ausgewogene Verteilung der Verformung über den gesamten Steg sicherzustellen.

Vorzugsweise verläuft die innere Trennfuge dabei im ersten Abschnitt in im wesentlichen konstanten Abstand von der Außenkontur, im Zwischenabschnitt entlang der Symmetrieachse und im zweiten Abschnitt im wesentlichen T-förmig. Hierdurch werden vorteilhafterweise wenigstens im Zwischenabschnitt und im zweiten Abschnitt der expansiblen Elemente Aussparungen in der Mantelfläche des Stents verhindert. Der Querbalken der T-förmigen Trennfuge im zweiten Abschnitt verläuft dabei vorzugsweise in wesentlichen parallel zur Stirnseite, d.h. der dem ersten Abschnitt abgewandten Seite des zweiten Abschnitts, um eine ausreichende Verformbarkeit des umlaufenden Steges in diesem Bereich sicherzustellen.

Bei einer besonders günstigen Variante weist die innere Trennfuge im ersten Abschnitt eine Unterbrechung auf, wodurch eine Verbindung zwischen dem umlaufenden Steg und dem davon begrenzten Bereich im Innern des expansiblen Elements entsteht. Hiermit wird vorteilhafterweise vermieden, daß im Innern des expansiblen Elements eine Aussparung entsteht. Es ist hierdurch in einfacher Weise möglich, die gesamte Mantelfläche des Stents aussparungsfrei zu gestalten.

Vorzugsweise ist die Unterbrechung der inneren Trennfuge im ersten Abschnitt der expansiblen Elemente im Bereich der Stirnseite, d.h. der dem zweiten Abschnitt abgewandten Seite des ersten Abschnitts angeordnet. Da die Unterbrechung im Bereich der Symmetrieachse des expansiblen Elements angeordnet ist, in dem die Verformung des umlaufenden Steges gering ist ergibt sich hierdurch zum einen bei der Expansion des Stents eine besonders gleichförmige, symmetrische Verformung des umlaufenden Steges, zum anderen wird die durch die Unterbrechung der Trennfuge entstehende Verbindung dort am wenigsten belastet.

Bei einer vorteilhaften Variante ist der jeweilige Verbindungssteg im an den ersten Abschnitt angrenzenden Bereich des Zwischenabschnitts mit dem expansiblen Element verbunden und durch eine Trennfuge vom jeweiligen ersten Abschnitt getrennt. Hierdurch ergibt sich eine besonders günstige Krafteinleitung in den umlaufenden Steg und damit auch eine besonders günstige Verformung des umlaufenden Steges bei der Expansion des Stents.

Bei einer weiteren vorteilhaften Ausführung des erfindungsgemäßen Stents ist der Anschluß des expansiblen Elements zu dem in Längsrichtung des Stents angrenzenden expansiblen Element an der Stirnseite des ersten Abschnittes angeordnet. Wie schon bei der Anordnung der Unterbrechung der inneren Trennfuge ergeben sich auch hier die Vorteile einer gleichmäßigen Verformung des umlaufenden Steges bei geringer Belastung des Anschlusses.

Bei einer günstigen Ausführung des erfindungsgemäßen Stents umfaßt der Anschluß wenigstens ein zwischen den Stirnseiten der ersten Abschnitte zweier in Längsrichtung des Stents aneinander angrenzender expansibler Elemente angeordnetes Dehnungselement, das durch wenigstens zwei im wesentlichen parallele und in Umfangsrichtung verlaufende Trennfugen als im wesentlichen S-förmiger Dehnungssteg ausgebildet ist. Dieser Dehnungssteg ermöglicht eine axiale Bewegung der beiden über den Anschluß miteinander verbundenen expansiblen Elemente zueinander. Hierdurch wird in einfacher Weise der Vorteil erzielt, daß zum einen eine eventuelle Verkürzung des Stents bei der Expansion kompensiert werden kann, zum anderen ist es hiermit möglich, den gesamten Stent im Bereich dieser Dehnungselemente, der einer definierten Knickung zu unterwerfen, wenn sich die Dehnungselemente aufgrund der Anordnung expansiblen Elemente auf einem gemeinsamen Ringabschnitt des Stents befinden. Hierdurch ist es möglich auch in gekrümmten Blutgefäßen vorhandene Stenosen erfolgreich durch das Expandieren von Stents therapieren zu können. Vorzugsweise erstrecken sich die Dehnungselemente dabei im nicht expandierten Zustand des Stents zur Bildung eines geschlossenen Ringbereiches auf der Mantelfläche des Stents jeweils in Umfangsrichtung bis zu den in Umfangsrichtung benachbarten Dehnungselementen.

Bei günstigen Ausführungen des erfindungsgemäßen Stents Stent sind zwei in Umfangsrichtung des Stents aneinandergrenzende expansible Elemente jeweils zum Mittelpunkt ihres Verbindungssteges zueinander punktsymmetrisch. Hierdurch ist eine besonders einfache Herstellbarkeit des Stents gewährleistet.

Durch den bei der Expansion des Stents gleichzeitig auftretenden Vorgang der Dehnung der expandierbaren Elemente in Umfangsrichtung des Stents und durch das Ausrichten der schrägstehenden Verbindungselement in Querrichtung werden die jeweils nicht miteinander verbundenen Gruppen von expansiblen Elementen so gegeneinander verschoben, daß diese Bewegung die Verkürzung des Stents durch Ausdehnung der expansiblen Elemente kompensiert.

Bei vorteilhaften Ausführungen des erfindungsgemäßen Stents weisen die Verbindungselemente bei nicht expandiertem Stent eine Neigung zur Umfangsrichtung von im wesentlichen 45° auf, da hierdurch eine besonders günstige Verformung der expansiblen Elemente erzielt wird.

Die in Querrichtung expansiblen Element wirken dabei besonders günstig mit den Verbindungselementen zusammen, wenn diese in die in Umfangsrichtung expandierbaren Elemente - bezogen auf deren lokale Richtung - unter einem Winkel von mehr als 45° einmünden.

Eine besonders günstige Konstruktion ergibt sich noch nicht nur durch die konstruktive Auslegung in Bezug auf eine möglichst große Festigkeit durch Erhöhung der Materialquerschnitte, sondern auch dadurch, daß die Form der umlaufenden Stege und Verbindungsbereiche im Hinblick auf die zu erwartenden Belastungen optimiert wird. Dies geschieht einerseits dadurch, daß die auftretenden maximalen Spannungen lokal minimiert werden, anderseits aber auch dadurch, daß die notwendigen Verformungen kontrolliert werden.

Diese Zusammenhänge sind in einer bereits früher eingereichten Patentanmeldung derselben Inhaberin beschrieben.

Vorzugsweise sind die Verbindungsbereiche zwischen dem expansiblen Element und den Verbindungsstegen bzw. dem Anschluß sowie Umlenkungspunkte des Kraftflusses im expansiblen Element bzw. im Dehnungssteg derart ausgebildet sind, daß die Materialspannungen bei der Verformung auch als Kerbspannung einen vorgegebenen Wert nicht überschreitet.

Um lokale spannungsfreie Verformungen lokal zu begünstigen, ist es weiter vorzugsweise vorgesehen, daß die Verbindungsbereiche zwischen dem expansiblen Element und den Verbindungsstegen bzw. dem Anschluß unter Vermeidung von sprungartigen Änderungen der Stegbreite ausgebildet sind. Hiermit läßt sich erreichen, daß die Materialspannungen insbesondere im Bereich von Kreuzungen oder Verzweigungen bei der Verformung auch als Kerbspannung einen vorgegebenen Wert nicht überschreitet.

Bei vorteilhaften Ausführungen weisen die Trennfugen in den Verbindungsbereichen zwischen dem expansiblen Element und den Verbindungsstegen bzw. dem Anschluß sowie an Umlenkungspunkten des Kraftflusses im expansiblen Element () bzw. im Dehnungssteg () entsprechende Krümmungsradien oder einen aufgeweiteten Endbereich () mit entsprechendem Öffnungsradius auf, um die Kerbspannungsempfindlichkeit der Bauteile an diesen Punkten durch die Ausrundungen zu reduzieren.

Ein örtliches Aufbrechen des Stents führt in nachteiliger Weise zu freien, relativ scharfkantigen Enden innerhalb der Raumkonfiguration des Stent, welche einerseits die Gefäßwandung durchbohren können oder andererseits den freien Querschnitt des Gefäßes durch ein Auslenken in die Blutbahn reduzieren.

Vorteilhafte Formgebungen sind durch eine Verrundung aller Verbindungspunkte der sich beim Expandieren des Stents relativ zueinander bewegenden Elemente gekennzeichnet und sichern in einfacher und zugleich vorteilhafter Weise, daß das Maß der lokalen Materialverformung an den hinsichtlich der auftretenden Spannungsbelastung kritischen Punkten der Stentkonstruktion beim Aufweiten des Stents durch gleichmäßige Verteilung der Verformungsarbeit einen minimalen Wert aufweist.

Ein bevorzugter Stent der vorstehenden Auslegung besteht aus Tantal als Werkstoff und ist mit einer Beschichtung aus amorphen Siliciumcarbid versehen.

Bei dem erfindungsgemäßen Verfahren zur Herstellung eines erfindungsgemäßen Stents werden die Trennfugen in der Mantelfläche des rohrförmigen Körpers durch wenigstens einen auf die Mantelfläche gerichteten Schneidstrahl erzeugt, der entlang der Trennfugen geführt wird. Dieses Verfahren zeichnet sich durch seine besondere Einfachheit und die erreichbare hohe Schnittpräzision aus. Durch direktes Schneiden der Trennfugen in die Mantelfläche des Stentrohlings kann der Stent in einem Arbeitsgang erzeugt werden. Eine anschließende weitere mechanische Bearbeitung des Stents ist nicht erforderlich, so daß das Risiko einer Beschädigung des Stents nach Herstellen der Stentstruktur minimiert ist.

Es ist beispielsweise möglich, einen Wasserstrahlschneidvorgang anzuwenden, um die Trennfugen zu erzeugen. Vorzugsweise wird als Schneidstrahl aber ein Laserstrahl verwendet wird, da hierbei zum einen eine sehr hohe Schnittpräzision erreichbar ist und zum anderen die auf den Stent wirkenden mechanischen Belastungen minimiert sind.

Bei einer bevorzugten Ausführung des Verfahrens weist die Vorschubgeschwindigkeit des Schneidstrahls beim Abfahren des Verlaufs der Trennfugen zwischen den aufgeweiteten Endbereichen der Trennfugen ausschließlich eine Geschwindigkeitskomponente in Richtung des Verlaufs der Trennfugen aufweist. Hierdurch wird eine kurze Bearbeitungszeit des Stents erzielt. Vorzugsweise entspricht die Vorschubgeschwindigkeit des Schneidstrahls im wesentlichen der maximalen Vorschubgeschwindigkeit des Schneidstrahls, bei der noch ein sauberer Schnittspalt in der Mantelfläche erzielt wird. Hiermit ergeben sich nicht nur minimierte Bearbeitungszeiten des Stents, es werden auch in vorteilhafter Weise besonders schmale Trennfugen hergestellt.

Bei vorteilhaften Ausführungen des Verfahrens weist die Vorschubgeschwindigkeit des Schneidstrahls zur Erzeugung der aufgeweiteten Endbereiche der Trennfugen zeitweise eine Geschwindigkeitskomponente quer zum Verlauf der Trennfugen auf. Vorzugsweise vollführt der Schneidstrahl dabei eine mäanderförmige oder eine kreis- oder ellipsenförmige Vorschubbewegung oder eine Kombination daraus.

Bei einer bevorzugten Ausführung des erfindungsgemäßen Verfahrens die werden die aufgeweiteten Endbereiche der Trennfugen durch eine Verringerung der Vorschubgeschwindigkeit des Schneidstrahls im Bereich der aufgeweiteten Endbereiche erzeugt. Hierdurch wird der Herstellungsaufwand für den Stent noch weiter reduziert, da der Schneidstrahl keine komplizierten Bahnbewegungen durchlaufen muß, sondern sich lediglich entsprechend langsamer fortbewegen muß, um einen entsprechend größeren Materialabtrag zu erzielen als bei normaler Vorschubgeschwindigkeit. Je geringer die Vorschubgeschwindigkeit des Schneidstrahls ist, desto größer ist der Materialabtrag und damit auch die Breite bzw. der Ausrundungsradius des aufgeweiteten Endbereiches. Bei einer besonders einfachen Variante werden die aufgeweiteten Endbereiche der Trennfugen durch das Verharren des Schneidstrahls in der entsprechenden Schneidposition erzeugt

Andere vorteilhafte Weiterbildungen der Erfindung sind in den Unteransprüchen gekennzeichnet bzw. werden nachstehend zusammen mit der Beschreibung bevorzugter Ausführungen der Erfindung anhand der Figuren näher dargestellt. Es zeigen:
- Figur 1: als bevorzugtes Ausführungsbeispiel der Erfindung einen Teil der Abwicklung der Mantelfläche eines nicht expandierten Stents,
- Figur 2: die Abwicklung eines Teils der Mantelfläche nach Figur 1 in teilexpandiertem Zustand, in vergrößertem Maßstab,
- Figur 3: die Abwicklung des Teils der Mantelfläche nach Figur 2 im vollständig expandierten Zustand,
- Figur 4: einen Ausschnitt aus der Mantelfläche nach Figur 1 in geknicktem, nicht expandiertem Zustand des Stents,
- Figur 5: einen Ausschnitt aus der nicht abgewickelten Mantelfläche nach Figur 1 in nicht expandiertem Zustand des Stents in perspektivischer Ansicht,
- Figur 6: einen Ausschnitt aus der abgewickelten, nicht expandierten Mantelfläche einer weiteren bevorzugten Ausführung des erfindungsgemäßen Stents in vergrößertem Maßstab sowie
- Figur 7: ein weiteres bevorzugtes Ausführungsbeispiel der Abwicklung der Mantelfläche eines nicht expandierten erfindungsgemäßen Stents.

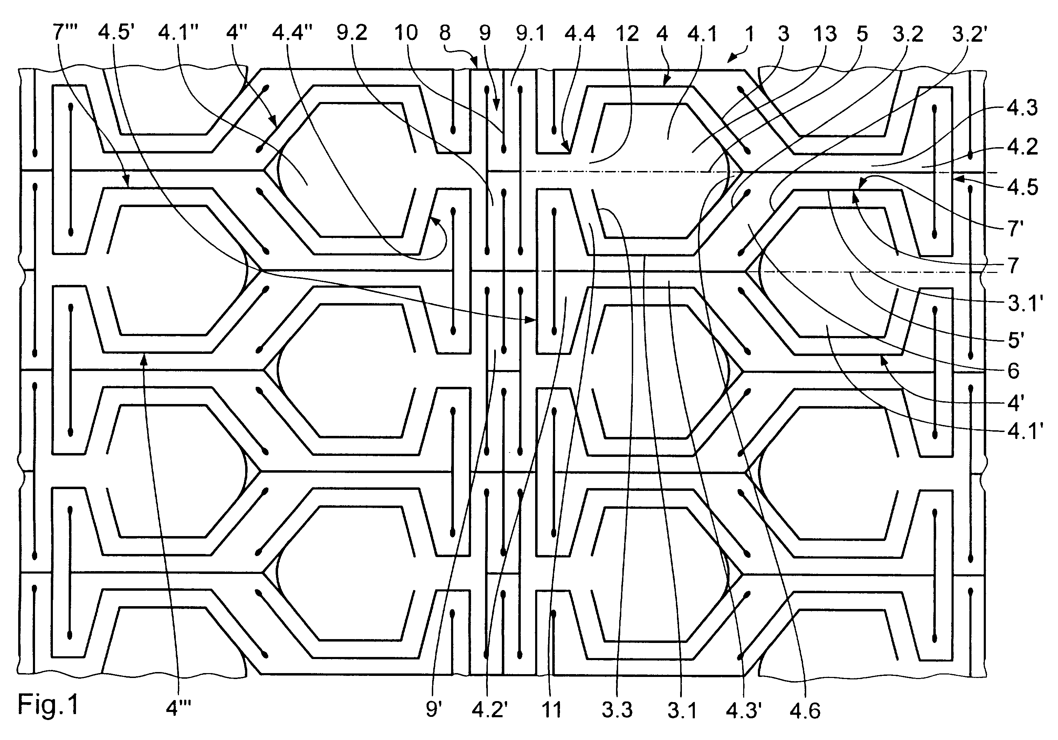

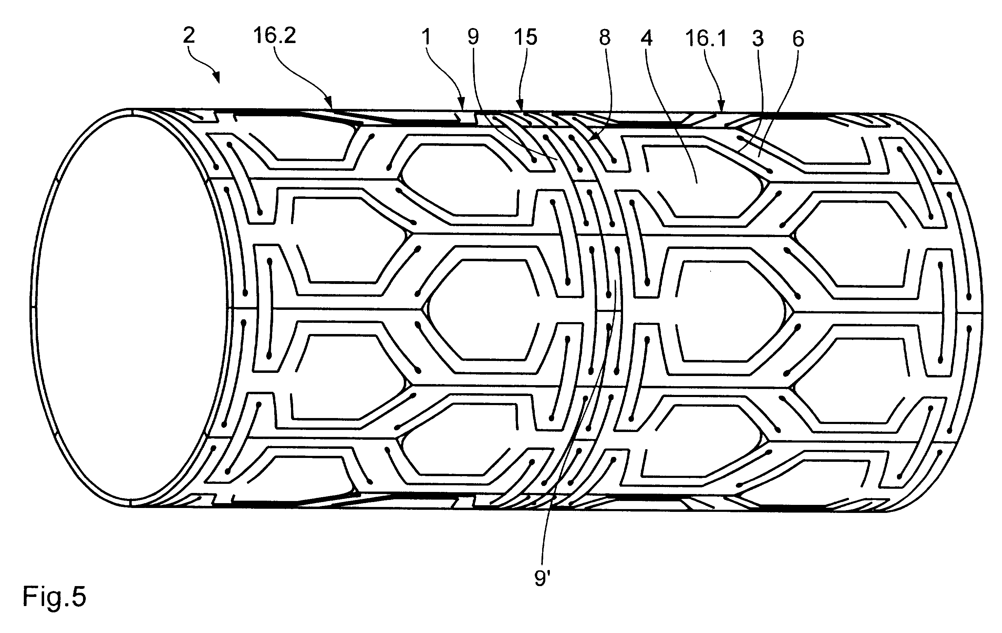

Figur 1 zeigt einen Teil der abgewichkelten Mantelfläche 1 eines Stents aus einem rohrförmigen Körper 2 (siehe Fig. 5). Die Mantelfläche 1 ist durch Trennfugen 3 in eine Vielzahl gleichartiger, stegartiger, sich in Längsrichtung des Stents erstreckender und in Umfangsrichtung des Stents expansibler Elemente 4 unterteilt. Die Trennfugen 3 weisen dabei eine im Verhältnis zur Stegbreite der expansilblen Elemente 4 wesentlich geringere Breite auf. Im gezeigten Beispiel wurden sie durch einen Laserstrahl in die Mantelfläche 1 geschnitten. Der Laserstrahl wurde dabei mit der maximalen Vorschubgeschwindigkeit entlang des Trennfugenverlaufs geführt, mit der noch ein sauberer Schnittspalt in der Mantelfläche 1 erzielbar ist.

Die expansilblen Elemente 4 sind zu ihrer Längsachse 5 symmetrisch. Sie weisen einen ersten Abschnitt 4.1 in Form eines unregelmäßigen Sechseckes sowie einen zweiten Abschnitt 4.2 auf, der die Kontur eines unregelmäßigen Fünfeckes besitzt und sich im wesentlichen in Umfangsrichtung des Stents, d.h. senkrecht zur Längsachse des expansiblen Elements 4 erstreckt. Der erste Abschnitt 4.1 und der zweite Abschnitt 4.2 sind über einen langgestreckten Zwischenabschnitt 4.3 miteinander verbunden.

Jeweils zwei in Umfangsrichtung des Stents benachbarte expansible Elemente 4, 4' sind über einen Verbindungssteg 6 miteinander verbunden. Die beiden expansible Elemente 4 und 4' sind dabei um 180° zueinander gedreht, wobei der erste Abschnitt 4.1 des ersten expansiblen Elements 4 in den Zwischenraum zwischen dem ersten Abschnitt 4.1' und dem zweiten Abschnitt 4.2' des zweiten expansiblen Elements 4' eingreift. Umgekehrt greift der erste Abschnitt 4.1' des zweiten expansiblen Elements 4' in den Zwischenraum zwischen dem ersten Abschnitt 4.1 und dem zweiten Abschnitt 4.2 des ersten expansiblen Elements 4 ein. Der Verbindungssteg 6 ist zwischen den beiden ersten Abschnitten 4.1 und 4.1' der beiden Elemente angeordnet. Er verläuft dabei unter einem Winkel von etwa 45° zur Umfangsrichtung des Stents.

Die Außenkonturen 7, 7' der expansiblen Elemente 4, 4' sind dabei so ausgebildet, daß die einander unmittelbar zugewandten Außenkonturbereiche der beiden Elemente 4, 4' durch dieselben Trennfugen 3.1, 3.1' begrenzt werden. So begrenzt die Trennfuge 3.1 sowohl den Teil der Außenkontur 7 des ersten expansiblen Elements 4 in dem Bereich, in dem der erste Abschnitt 4.1 des ersten expansiblen Elements 4 an den zweiten Abschnitt 4.2' und den Zwischenabschnitt 4.3' des zweiten expansiblen Elements 4' angrenzt, als auch den zweiten Abschnitt 4.2' und den Zwischenabschnitt 4.3' des zweiten expansiblen Elements 4' selbst. Analoges gilt für die Trennfuge 3.1'.

Der Verbindungssteg 6 ist über seine gesamte Länge seitlich von den Trennfugen 3.2 bzw. 3.2' begrenzt, die jeweils in diesem Bereich die Außenkontur 7 bzw. 7' des ersten expansilblen Elementes 4 bzw. des zweiten expansilblen Elementes 4' begrenzt. Er ist jeweils an den Zwischenabschnitten 4.3, 4.3' mit den expansiblen Elementen 4, 4' verbunden.

Aufgrund dieser Anordnung bestehen auf der Mantelfläche 1 des Stents keinerlei Lücken zwischen in Umfangsrichtung benachbarten expansiblen Elementen 4, 4'.

An der Stirnseite 4.4 des ersten Abschnittes 4.1 des expansiblen Elements 4 ist ein Anschluß 8 angeordnet, über den das Element 4 mit einem in Längsrichtung des Stents daran angrenzenden 4" verbunden ist. Dieser Anschluß 8 umfaßt dabei ein Dehnungselement 9, das durch entsprechende Trennfugen 10 in zwei zueinander zur Längsachse 5 spiegelsymmetrische Dehnungsstegen 9.1 und 9.2 ausgebildet ist, die im wesentlichen määnderförmig - mit enem doppelten S-Schlag - verlaufen.

Die Außenkonturen 7', 7"' der expansiblen Elemente 4', 4"' sind im Bereich des Anschlusses 8 so ausgebildet, daß auch in Längsrichtung des Stents keine Aussparungen zwischen den einzelnen Bestandteilen der Mantelfläche 1 entstehen.

Das Dehnungselement 9 erstreckt sich in Umfangsrichtung des Stents bis zu den angrenzenden Dehnungselementen hin, wobei die Kontur der Dehnungselemente so ausgebildet ist, daß auch in Umfangsrichtung keine Aussparungen in der Mantelfläche 1 entstehen.

Im Innern des expansiblen Elements 4 ist eine innere Trennfuge 3.3 angeordnet, die entlang der Außenkontur 7 verläuft und durch die ein schmaler, entlang der Außenkontur 7 umlaufender Steg 11 gebildet ist. Die innere Trennfuge verläuft im ersten Abschnitt 4.1 im wesentlichen in konstantem Abstand zur Außenkontur 7, wobei sie im Bereich der Stirnseite 4.4 des ersten Abschnitts 4.1 eine Unterbrechung 12 aufweist, durch die das durch die innere Trennfuge 3.3 begrenzte Mantelstück 13 mit dem umlaufenden Steg 11 verbunden ist. Hierdurch wird verhindert, daß im Bereich des Mantelstücks 13 eine Aussparung in der Mantelfläche 1 entsteht. Die infolge der Verrundung des Mantelstücks 13 entsteht lediglich eine vernachlässigbar kleine Aussparung 4.6 in der Mantelfläche 1. Im Bereich des Zwischenabschnitts 4.3 verläuft die innere Trennfuge 3.3 auf der Längsachse 5. Im zweiten Abschnitt 4.2 verläuft die innere Trennfuge 3.3 T-förmig, wobei der Querbalken des T parallel zur Stirnseite 4.5 des zweiten Abschnitts 4.2 verläuft. Hierdurch ist sichergestellt, daß sich der durch die innere Trennfuge 3.3 gebildete umlaufende Steg 11 bei der Verformung während der Expansion des Stents im Bereich der Stirnseite 4.5 gleichmäßig verformen kann, wodurch die an den Endpunkten der innere Trennfuge 3.3 auftretenden Spannungen bei der Verformung reduziert werden.

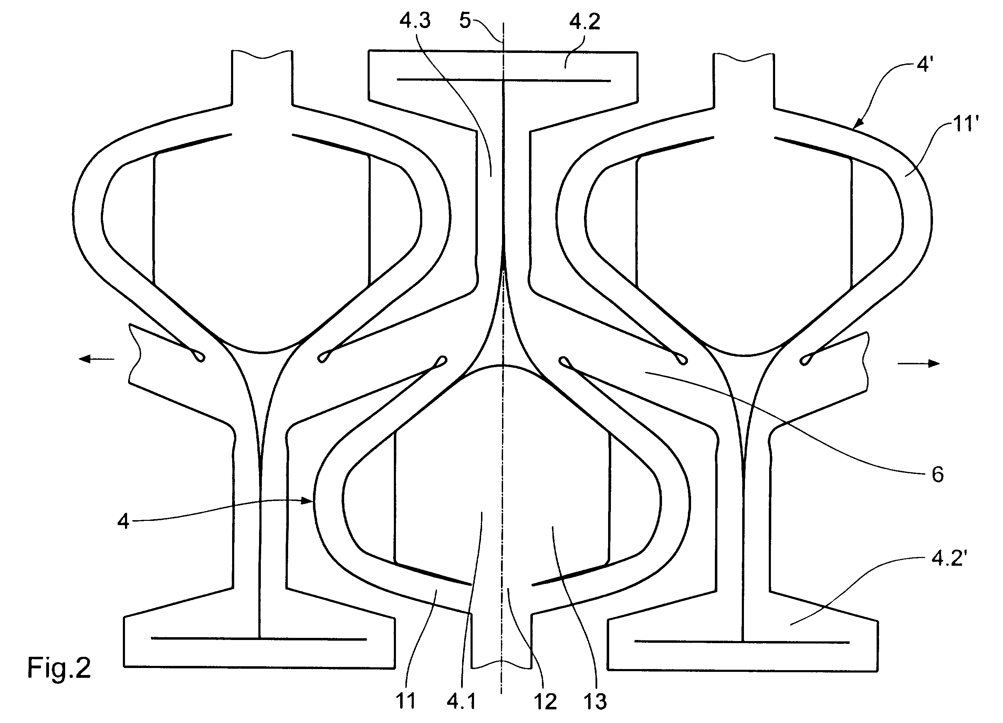

Es versteht sich, daß die Unterbrechung 12 im Hinblick auf die ansonsten vollständig geschlossene Mantelfläche 1 nicht unbedingt erforderlich ist, d.h. daß eine derartige oder andere Aussparungen im wesentlichen vernachlässigbaren Ausmaßes im Rahmen der Erfindung ohne weiteres tragbar wären. Figur 2 zeigt einen Ausschnitt aus der Mantelfläche 1 des Stents im teilexpandierten Zustand. Bei der Expansion des Stents entfernen sich die expansiblen Elemente 4, 4' in Umfangsrichtung voneinander. Über den Verbindungssteg 6, über den sie miteinander verbunden sind werden dabei die Verformungskräfte in die umlaufenden Stege 11, 11' der expansiblen Elemente 4, 4' eingeleitet. Der Neigungswinkel des Verbindungssteges 6 zur Umfangsrichtung verringert sich dabei, so daß ein neben einer Kraft in Umfangsrichtung auch ein Biegemoment in den umlaufenden Steg 11 eingeleitet wird. Dieses Biegemoment bewirkt, daß sich zunächst im wesentlichen der im ersten Abschnitt 4.1 verlaufende Teil des umlaufenden Steges 11 verformt. Dieser wölbt sich in Umfangsrichtung zunächst stark aus, wodurch der erste Abschnitt 4.1 stark verkürzt wird. Durch die starke Verkürzung des ersten Abschnitts 4.1 wird erreicht, daß sich der erste Abschnitt 4.1 des ersten expansiblen Elements 4 und der zweite Abschnitt 4.2' des zweiten expansiblen Elements 4' bei der Expansion nicht gegenseitig behindern, obwohl sich diese beiden Abschnitte bei der Expansion des Stents durch die Winkeländerung des Verbindungssteges 6 in Längsrichtung aufeinander zubewegen. Diese Längsbewegung bewirkt wiederum, daß Verkürzungen des Stents bei dessen Expansion vermieden werden.

Das Mantelstück 13 das bei der Verformung des umlaufenden Steges 11 in Richtung des Zwischenabschnittes 4.3 verschoben wird, unterstützt dabei die gleichmäßige und schonende Verformung des umlaufenden Steges 11.

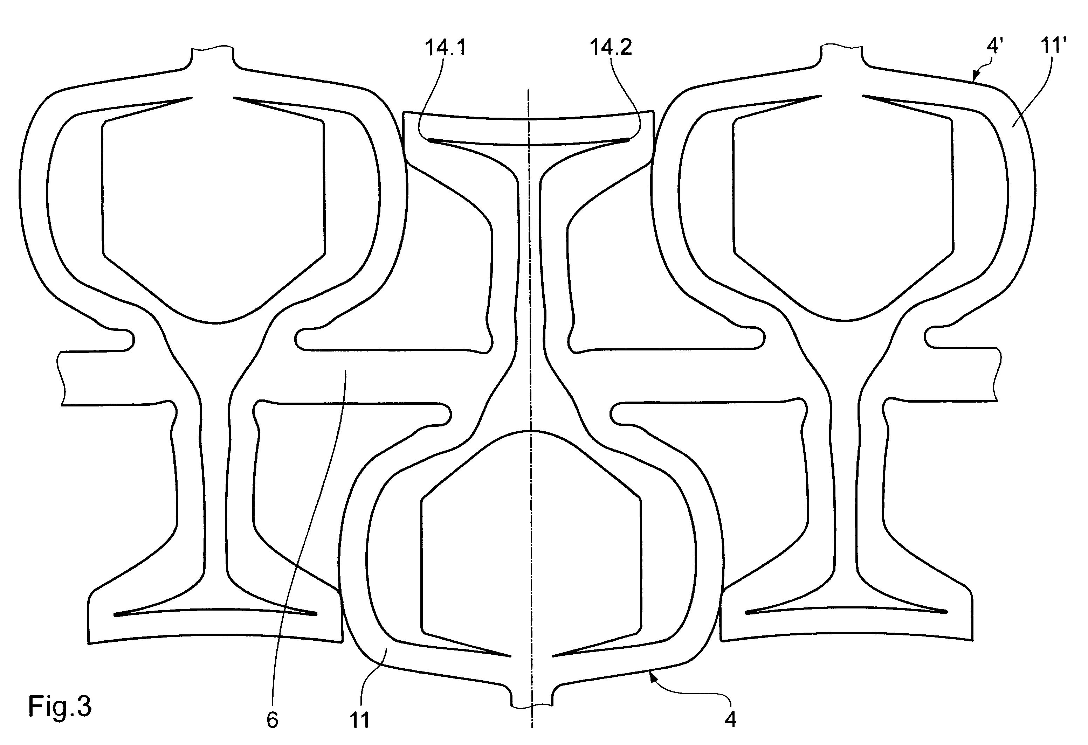

Figur 3 zeigt den Teil der Mantelfläche 1 aus Fig. 2 im vollständig expandierten Zustand. Der Verbindungssteg 6 ist dabei nunmehr vollständig in Umfangsrichtung orientiert.

Die innere Trennfuge 3.3 ist über ihren gesamten Bereich relativ gleichmäßig geöffnet, so daß auch die Verformung über den gesamten umlaufenden Steg 11 relativ gleichmäßig verteilt ist. Insbesondere in den besonders kerbempfindlichen Endpunkten 14.1 und 14.2 der inneren Trennfuge 3.3 im zweiten Abschnitt 4.2 treten dabei keine unzulässig hohen Spannungen auf.

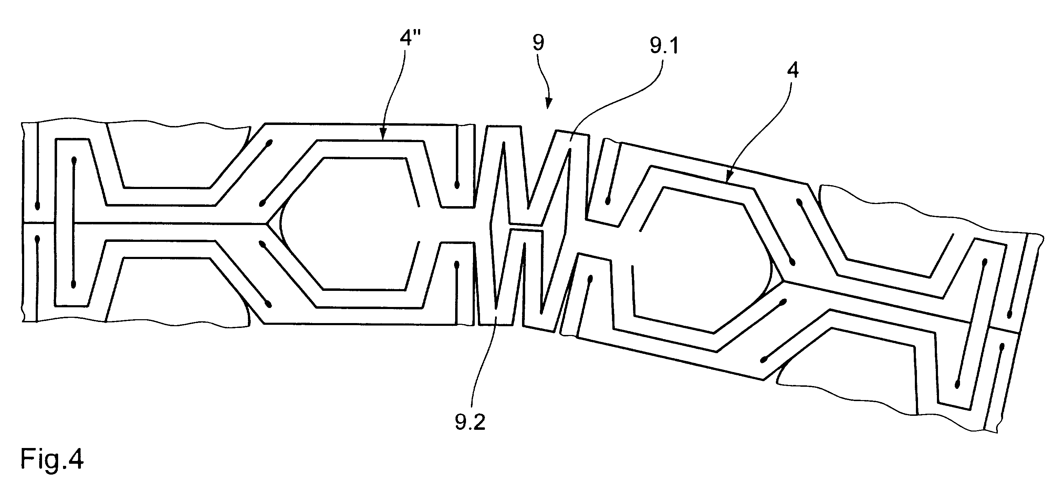

Figur 4 zeigt schematisch die Wirkungsweise der Dehnungselemente 9 bei einer Knickung des gesamten Stents in einer zur Zeichenebene parallelen Ebene, wobei die Längschse des Stents hinter der Zeichenebene liegt. Die Dehnungsstege 9.1 und 9.2 können dabei zunächst axial in Längsrichtung des Stents auseinandergezogen. Je nach Knickwinkel wird dann der Dehnungssteg 9.1 noch weiter gedehnt, während der Dehnungssteg 9.2 wieder komprimiert wird. Es ist allerdings auch möglich, daß die Dehnungsstege 9.1 und 9.2 von vornherein nur unterschiedlich stark in Längsrichtung gedehnt werden.

Figur 5 zeigt die Mantelfläche 1 nach Figur 1 in nicht expandiertem Zustand des Stents. Der rohrförmige Körper 2 weist eine geschlossene Mantelfläche 1 auf, die durch Trennfugen 3 in expansible Elemente 4, Verbindungsstege 6 und Anschüsse 8 unterteilt ist. Die Dehnungselemente 9 sind dabei zu einem geschlossenen Ringbereich 15 angeordnet. Die in Umfangsrichtung benachbarten expansiblen Elemente 4 bilden ebenfalls geschlossene Ringbereiche 16.1, 16.2.

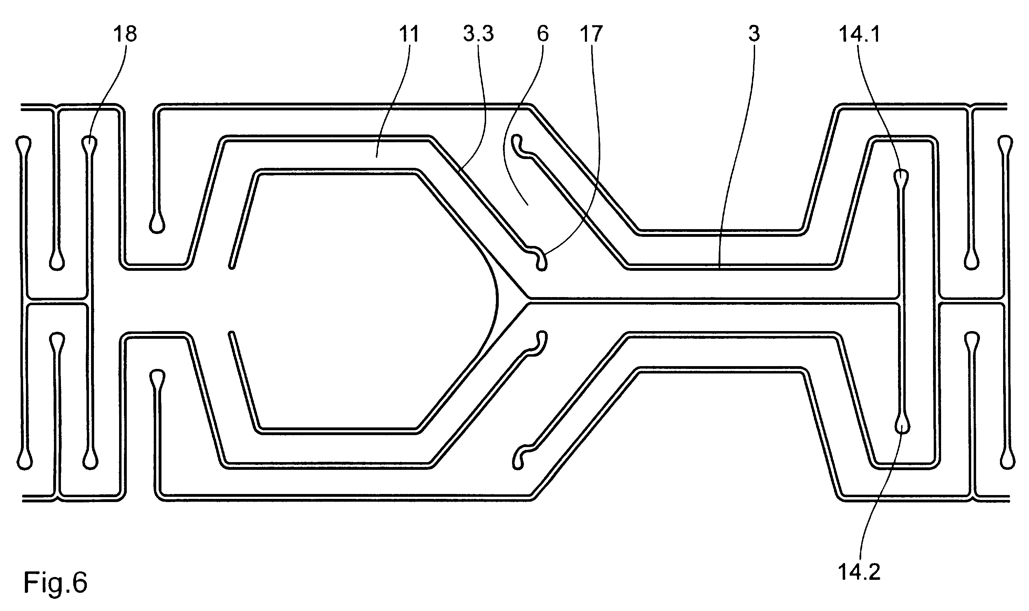

Figur 6 zeigt einen vergrößerten Ausschnitt aus der Mantelfläche einer weiteren Ausfuhrungsform eines erfindungsgemäßen Stents. Die Endpunkte 14.1, 14.2, 17, 18 sämtlicher Trennfugen 3 weisen dabei Aufweitungen und Ausrundungen auf, um die Kerbspannungsempfindlichkeit der Bauteile an diesen Stellen zu reduzieren. Die Tropfenform wurde dabei dadurch erzeugt, daß die Vorschubgeschwindigkeit des zur Herstellung der Trennfugen verwendeten Laserstrahls bei der Annäherung an den Endpunkt einer Trennfuge gleichmäßig bis zum Stillstand verringert wurde, so daß sich ein immer breiterer Schnittspalt und schließlich die Ausrundung am Endpunkt ergaben.

Weiterhin mündet der Steg im Bereich des Endpunktes 17 der Trennfuge 3.3 durch die Gestaltung des Endverlaufes der Trennfuge 3.3 in einem Winkel von mehr als 45° (etwa 90°) in den umlaufenden Steg 11. Hierdurch wird an dieser Stelle eine günstige und schonende Krafteinleitung in den umlaufenden Steg 11 erzielt.

Verzweigungen oder Umlenkungen von Stegen sind unter Vermeidung von sprungartigen Änderungen der Stegbreite derart gestaltet, daß die Materialspannungen insbesondere im Bereich der Verzweigung oder Umlenkung bei der Verformung auch als Kerbspannung einen vorgegebenen Wert nicht überschreitet.

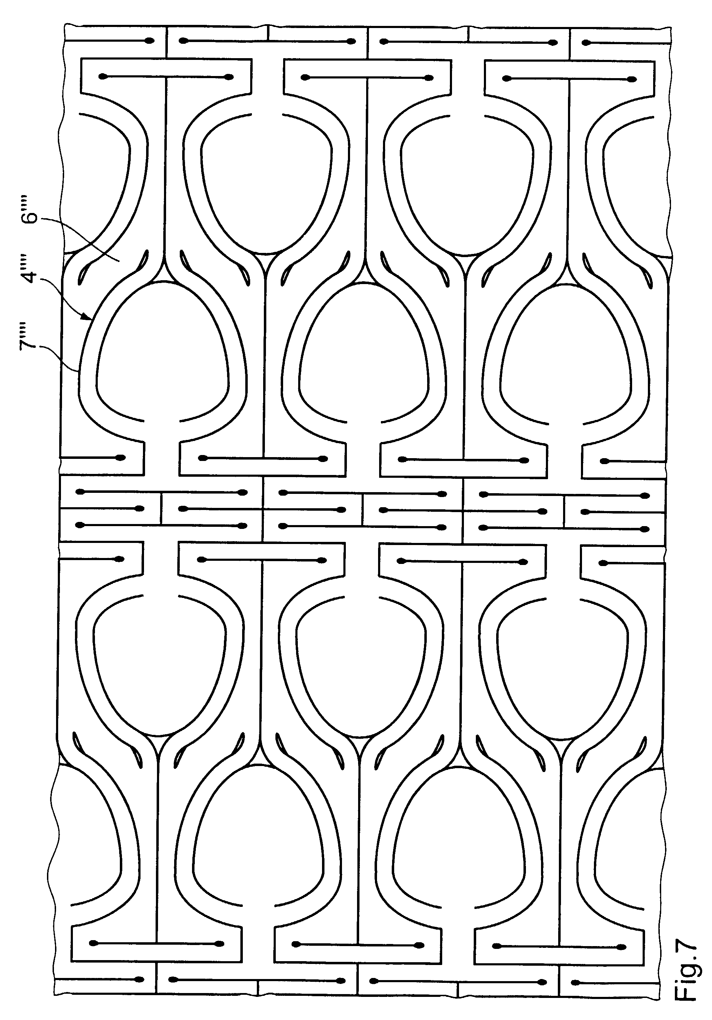

Figur 7 zeigt ein weiteres bevorzugtes Ausführungsbeispiel der Abwicklung der Mantelfläche eines nicht expandierten erfindungsgemäßen Stents. Bei dieser Ausführung weisen die expansiblen Elemente 4"" und die übrigen Bestandteile des Stents im wesentlichen dieselben Merkmale und Eigenschaften, wie für die vorstehend beschriebenen Ausführungen auf. Lediglich die Außenkontur 7"" der expansiblen Elemente 4"" unterscheidet sich durch ihre stark gerundeten Formen, die zu einer noch verbesserten Krafteinleitung und Verteilung der Verformung über die expansiblen Elemente 4"" führt.

Die vorstehend beschriebene Ausbildungen der Stents gestatten ein Expandieren der rohrförmigen Stents, ohne daß es an den Verbindungspunkten zur Ausbildung von zur Zerstörung von Stegbereichen führenden Extremwerten der Kerbspannung kommt.

Die hier dargestellte Stents bestehen aus Tantal, Titan oder einer anderen biokompatiblen Materiallegierung, woraus eine gute Körperverträglichkeit und eine ausgezeichnete Verformbarkeit resultiert. Eine Mikrobeschichtung aus amorphem Siliciumcarbid wirkt einer Thrombenbildung entgegen.

Die Erfindung beschränkt sich in ihrer Ausführung nicht auf die vorstehend angegebenen bevorzugten Ausführungsbeispiele. Vielmehr ist eine Anzahl von Varianten günstig, welche von der dargestellten Lösung auch bei grundsätzlich anders gearteten Ausführungen Gebrauch macht.

## Patentansprüche

1. Stent, insbesondere Koronarstent, bestehend aus mindestens einem dünnwandigen, rohrförmigen Körper (2), dessen Mantelfläche (1) in stegartige, sich in Längsrichtung des Stents erstreckende und in Umfangsrichtung des Stents expansible Elemente (4, 4', 4", 4"', 4"") unterteilt ist, die über jeweils wenigstens einen Verbindungssteg (6, 6') mit den beiden in Umfangsrichtung des Stents benachbarten expansiblen Elementen (4, 4', 4", 4"', 4"") und über einen stirnseitigen Anschluß (8) mit der Stirnseite (4.4') eines in Längsrichtung des Stents angrenzenden expansiblen Elements (4, 4', 4", 4"', 4"") verbunden sind,
**dadurch gekennzeichnet,**
daß die Mantelfläche (1) im nicht expandierten Zustand im wesentlichen frei von Ausnehmungen ist und durch Trennfugen (3) unterteilt ist, die im Verhältnis zur Stegbreite der expansiblen Elemente (4, 4', 4", 4"', 4"") eine wesentlich geringere Breite aufweisen.

2. Stent nach Anspruch 1, **dadurch gekennzeichnet,** daß die Trennfugen (3) eine Breite aufweisen, die im wesentlichen der Breite eines durchgängig separierenden Schnittspaltes beim Durchtrennen der Mantelfläche (1) mittels eines Schneidstrahls, insbesondere eines Laserstrahls entspricht.

3. Stent nach Anspruch 2, **dadurch gekennzeichnet,** daß die Trennfugen (3) eine Breite aufweisen, die im wesentlichen der erzielbaren minimalen Breite eines durchgängig separierenden Schnittspaltes beim Durchtrennen der Mantelfläche (1) mittels eines Schneidstrahls, insbesondere eines Laserstrahls entspricht.

4. Stent nach einem der vorhergehenden Ansprüche, **dadurch gekennzeichnet,** daß die Verbindungsstege (6) sowohl zur Längs- als auch zur Umfangsrichtung des Stents geneigt verlaufen und im wesentlichen über ihre gesamte Länge seitlich jeweils von der Trennfuge (3.2, 3.2') begrenzt sind, die in diesem Bereich die Außenkontur des jeweiligen expansiblen Elements (4, 4') begrenzt.

5. Stent nach einem der vorhergehenden Ansprüche, **dadurch gekennzeichnet,** daß die Außenkontur (7, 7') der expansiblen Elemente (4, 4') zu ihrer Längsachse (5, 5') symmetrisch ist und einen sich sowohl in Längs- als auch in Umfangsrichtung erstreckenden flächigen ersten Abschnitt (4.1, 4.1') sowie einen sich wenigstens im wesentlichen in Umfangsrichtung erstreckenden zweiten Abschnitt (4.2, 4.2') aufweist, die durch einem sich im wesentlichen in Längsrichtung erstreckenden Zwischenabschnitt (4.3, 4.3') verbunden sind.

6. Stent nach Anspruch 5, **dadurch gekennzeichnet,** daß zwei in Umfangsrichtung des Stents aneinandergrenzende expansible Elemente (4, 4') jeweils um 180° zueinander gedreht sind und jeweils der erste Abschnitt (4.1, 4.1') des einen Elements (4, 4') in den Zwischenraum zwischen dem ersten Abschnitt (4.1', 4.1) und dem zweiten Abschnitt (4.2', 4.2) des anderen Elements (4', 4) eingreift sowie der Verbindungssteg (6) zwischen den beiden ersten Abschnitten (4.1, 4.1') angeordnet ist und die einander unmittelbar zugewandten Außenkonturbereiche der beiden Elemente (4, 4') durch dieselben Trennfugen (3.1, 3.1') begrenzt sind.

7. Stent nach einem der vorhergehenden Ansprüche, **dadurch gekennzeichnet,** daß zur Ausbildung eines entlang der Außenkontur (7, 7') umlaufenden Steges (11, 11') geringer Breite im Innern der expansiblen Elemente (4, 4') eine innere Trennfuge (3.3, 3.3') vorgesehen ist, die zumindest abschnittsweise in im wesentlichen konstanten Abstand von der Außenkontur (7, 7') verläuft.

8. Stent nach Anspruch 7, **dadurch gekennzeichnet,** daß die innere Trennfuge (3.3, 3.3') im ersten Abschnitt (4.1, 4.1') in im wesentlichen konstanten Abstand von der Außenkontur (7, 7'), im Zwischenabschnitt (4.3, 4.3') entlang der Symmetrieachse (5, 5') und im zweiten Abschnitt (4.2', 4.2) im wesentlichen T-förmig verläuft.

9. Stent nach einem der Ansprüche 7 oder 8, **dadurch gekennzeichnet,** daß die innere Trennfuge (3.3) im ersten Abschnitt (4.1) eine Unterbrechung (12) aufweist.

10. Stent nach Anspruch 9, **dadurch gekennzeichnet,** daß die Unterbrechung (12) der inneren Trennfuge (3.3) im ersten Abschnitt (4.1) des expansiblen Elements (4) im Bereich der Stirnseite (4.4) des ersten Abschnitts (4.1) angeordnet ist.

11. Stent nach einem der Ansprüche 5 bis 10, **dadurch gekennzeichnet,** daß der jeweilige Verbindungssteg (6) im an den ersten Abschnitt (4.1, 4.1') angrenzenden Bereich des Zwischenabschnitts (4.3, 4.3') mit dem expansiblen Element (4, 4') verbunden ist und durch eine Trennfuge (3.2, 3.2') vom jeweiligen ersten Abschnitt (4.1, 4.1') getrennt ist.

12. Stent nach einem der vorhergehenden Ansprüche, **dadurch gekennzeichnet,** daß der Anschluß (8) an der Stirnseite (4.4) des ersten Abschnittes (4.1) angeordnet ist.

13. Stent nach einem der vorhergehenden Ansprüche, **dadurch gekennzeichnet,** daß der Anschluß (8) wenigstens ein zwischen den Stirnseiten (4.4, 4.4") der ersten Abschnitte (4.1, 4.1") zweier in Längsrichtung des Stents aneinander angrenzender expansibler Elemente (4, 4") angeordnetes Dehnungselement (9) umfaßt, das durch wenigstens zwei im wesentlichen parallele und in Umfangsrichtung verlaufende Trennfugen (10) als im wesentlichen S-förmig verlaufender Dehnungssteg (9.1, 9.2) ausgebildet ist.

14. Stent nach Anspruch 13, **dadurch gekennzeichnet,** daß sich die Dehnungselemente (9) im nicht expandierten Zustand des Stents zur Bildung eines geschlossenen Ringbereiches (15) jeweils in Umfangsrichtung bis zu den jeweils in Umfangsrichtung benachbarten Dehnungselementen (9') erstrekken.

15. Stent nach einem der vorhergehenden Ansprüche, **dadurch gekennzeichnet,** daß zwei in Umfangsrichtung des Stents aneinandergrenzende expansible Elemente (4, 4') jeweils zum Mittelpunkt ihres Verbindungssteges (6) zueinander punktsymmetrisch sind.

16. Stent nach einem der vorhergehenden Ansprüche, **dadurch gekennzeichnet,** daß die Verbindungsstege (6) bei nicht expandiertem Stent eine Neigung zur Umfangsrichtung von im wesentlichen 45° aufweisen.

17. Stent nach einem der vorhergehenden Ansprüche, **dadurch gekennzeichnet,** daß die Verbindungsstege (6) in die expansiblen Elemente (4, 4') unter einem Winkel von mehr als 45° einmünden.

18. Stent nach einem der vorhergehenden Ansprüche, **dadurch gekennzeichnet,** daß die expansiblen Elemente (4, 4') derart geformt sind, daß die Verformung, welcher der umlaufende Steg (11, 11') zwischen Verbindungsbereichen mit anderen expansiblen Elementen (4, 4') bei der Expansion insgesamt unterworfen wird, im wesentlichen minimiert ist.

19. Stent nach einem der vorhergehenden Ansprüche, **dadurch gekennzeichnet,** daß die expansiblen Elemente (4, 4') derart geformt sind, daß die Biegeverformung, der ein umlaufender Steg (11, 11') bei der Expansion innerhalb der rohrförmigen Körpers (2) unterworfen ist und die sich ergibt aus dem Integral der örtlichen Winkeländerungen bei Verformung, ermittelt über die Länge des jeweiligen umlaufenden Steges (11, 11') zwischen angrenzenden Verbindungsbereichen mit anderen expansiblen Elementen (4, 4'), sich in der Weise über die Länge des umlaufenden Steges (11, 11') verteilt, daß sie lokal einen vorgegebenen Wert nicht überschreitet.

20. Stent nach einem der vorangehenden Ansprüche, **dadurch gekennzeichnet,** daß die auf eine Länge von einem Fünftel eines umlaufenden Steges (11, 11') entfallende Biegeverformung nicht größer ist als ein Viertel der gesamten Biegeverformung, der dieser unterworfen ist

21. Stent nach vorangehenden Ansprüche, **dadurch gekennzeichnet,** daß die Verbindungsbereiche zwischen dem expansiblen Element (4, 4') und den Verbindungsstegen (6) bzw. dem Anschluß (8) unter Vermeidung von sprungartigen Änderungen der Stegbreite ausgebildet sind.

22. Stent nach einem der vorangehenden Ansprüche, **dadurch gekennzeichnet,** daß die Verbindungsbereiche zwischen dem expansiblen Element (4, 4') und den Verbindungsstegen (6) bzw. dem Anschluß (6) sowie Umlenkungspunkte des Kraftflusses im expansiblen Element (4, 4') bzw. im Dehnungssteg (9.1, 9.2) derart ausgebildet sind, daß die Materialspannungen bei der Verformung auch als Kerbspannung einen vorgegebenen Wert nicht überschreitet.

23. Stent nach Anspruch 22, **dadurch gekennzeichnet,** daß die Trennfugen (3) in den Verbindungsbereichen zwischen dem expansiblen Element (4, 4') und den Verbindungsstegen (6) bzw. dem Anschluß (8) sowie an Umlenkungspunkten des Kraftflusses im expansiblen Element (4, 4') bzw. im Dehnungssteg (9.1, 9.2) entsprechende Krümmungsradien oder einen aufgeweiteten Endbereich (14.1, 14.2, 17, 18) mit entsprechendem Öffnungsradius aufweisen.

24. Stent nach einem der vorangehenden Ansprüche, **dadurch gekennzeichnet,** daß als Werkstoff Titan, Tantal oder ein anderes biokompatibles Metall bzw. eine entsprechende biokompatible Metallegierung vorgesehen ist.

25. Stent nach Anspruch 24, **dadurch gekennzeichnet,** daß eine Beschichtung aus amorphem Siliciumcarbid vorgesehen ist.

26. Verfahren zur Herstellung eines Stents nach einem der vorhergehenden Ansprüche, **dadurch gekennzeichnet,** daß die Trennfugen (3) in der Mantelfläche (1) des rohrförmigen Körpers (2) durch wenigstens einen auf die Mantelfläche (1) des rohrförmigen Körpers (2) gerichteten Schneidstrahl erzeugt werden, der entlang des Verlaufs der Trennfugen (3) geführt wird.

27. Verfahren nach Anspruch 26, **dadurch gekennzeichnet,** daß als Schneidstrahl ein Laserstrahl verwendet wird.

28. Verfahren nach Anspruch 26 oder 27, **dadurch gekennzeichnet,** daß die Vorschubgeschwindigkeit des Schneidstrahls beim Abfahren des Verlaufs der Trennfugen (3) zwischen den aufgeweiteten Endbereichen (14.1, 14.2, 17, 18) der Trennfugen (3) ausschließlich eine Geschwindigkeitskomponente in Richtung des Verlaufs der Trennfugen (3) aufweist.

29. Verfahren nach einem der Ansprüche 26 bis 28, **dadurch gekennzeichnet,** daß die Vorschubgeschwindigkeit des Schneidstrahls im wesentlichen der maximalen Vorschubgeschwindigkeit des Schneidstrahls entspricht, bei der noch ein sauberer Schnittspalt in der Mantelfläche (1) erzielt wird.

30. Verfahren nach einem der Ansprüche 26 bis 29, **dadurch gekennzeichnet,** daß die Vorschubgeschwindigkeit des Schneidstrahls zur Erzeugung der aufgeweiteten Endbereiche (14.1, 14.2, 17, 18) der Trennfugen (3) zeitweise eine Geschwindigkeitskomponente quer zum Verlauf der Trennfugen (3) aufweist.

31. Verfahren nach Anspruch 30, **dadurch gekennzeichnet,** daß der Schneidstrahl eine mäanderförmige und/oder eine kreis- oder ellipsenförmige Vorschubbewegung ausführt.

32. Verfahren nach Anspruch 26 bis 29, **dadurch gekennzeichnet,** daß die aufgeweiteten Endbereiche (14.1, 14.2, 17, 18) der Trennfugen (3) durch Verringerung der Vorschubgeschwindigkeit des Schneidstrahls im Bereich der aufgeweiteten Endbereiche (14.1, 14.2, 17, 18) erzeugt werden.

33. Verfahren nach Anspruch 32, **dadurch gekennzeichnet,** daß die aufgeweiteten Endbereiche (14.1, 14.2, 17, 18) der Trennfugen (3) durch Verharren des Schneidstrahls in der entsprechenden Schneidposition erzeugt werden.
